# FASCICULE DE BREVET EUROPEEN

(11) **EP 2 925 629 B1**
(45) Date de publication et mention de la délivrance du brevet: **12.04.2017**
(21) Numéro de dépôt: 13808122.9
(22) Date de dépôt: 27.11.2013
(51) Int. Cl.: B65D 75/32, B65D 75/58, B65D 51/32, A45D 34/04, A61F 13/40, B65D 77/20, A45D 40/00, A61M 35/00, B65D 75/36

(54) **DISPOSITIF POUR CONTENIR, DISTRIBUER ET APPLIQUER SUR UN SUPPORT, UN CONTENU SOUS FORME DE LIQUIDE, GEL, CRÈME PÂTE**
VORRICHTUNG ZUR AUFNAHME, AUFGABE UND ZUM AUFBRINGEN VON EINEM INHALT IN FORM EINER FLÜSSIGKEIT, EINES GELS ODER EINER CREME AUF EINEM TRÄGER
DEVICE FOR CONTAINING, DISTRIBUTING AND APPLYING CONTENTS IN THE FORM OF A FLUID, GEL OR CREAM ON A SUPPORT

(30) Priorité: 27.11.2012 FR 1261318
(43) Date de publication de la demande: 07.10.2015
(73) Titulaire: Socoplan, 79100 Saint Jean de Thouars (FR)
(72) Inventeur: MOUSSION, Philippe, 79100 Misse (FR); ALLAIN, Carl, 94170 Le Perreux sur Marne (FR)
(74) Mandataire: Derambure Conseil
(86) Numéro de dépôt international: PCT/FR2013/052876
(87) Numéro de publication internationale: WO 2014/083279

(56) Documents cités:
- DE-A1- 3 122 237
- DE-A1-102009 029 941

## Description

L'invention a pour objet un dispositif pour contenir, distribuer et appliquer sur une surface d'application, un contenu sous forme de liquide, gel, crème, pâte, ce dispositif étant soit à l'état fermé / associé avant que de pouvoir être amené à l'état ouvert / dissocié pour son utilisation, et où, selon les situations, il est empli ou vide de contenu.

Le domaine de l'invention est celui des dispositifs à usage unique pour une dose de contenu en quantité pouvant être appropriée pour un usage unique, en particulier une quantité comprise entre 0,5 ml et 10 ml, qui présentent, à l'état ouvert / dissocié, une partie d'utilisation et une partie jetable qui ne peut plus être remise en place sur la partie d'utilisation.

Le domaine de l'invention est également celui d'une application de contenu précise quant à la taille, la forme, la limite, et faible quant à la longueur, la surface, la quantité, le contenu étant sous forme de liquide, de gel, de crème, de pâte, et apte à pouvoir être appliqué comme avec un pinceau. Un tel contenu est par exemple un produit cosmétique, un produit de soin corporel, un produit d'hygiène corporelle, un médicament, un produit d'entretien, un produit de décoration, un produit d'écriture, un produit de masquage, une peinture, une encre, une laque, un vernis.

Le domaine de l'invention est également celui des dispositifs portatifs et nomades, légers et de petite taille - telle que par exemple de l'ordre de quelques centimètres pour sa plus grande longueur -, pouvant être rangés dans une poche, un sac, un vide-poche, un tiroir.

L'invention est adaptée au cas d'un dispositif nécessitant un conteneur accessoire ou complémentaire pour un contenu accessoire ou complémentaire et au cas d'un dispositif pouvant être agencé avec un ou plusieurs autres dispositifs analogues de sorte à former une plaque de dispositifs.

Le document WO 2010/133778 décrit un conditionnement non-rebouchable comprenant une coque rigide déformable sous l'action d'une pression de l'utilisateur, formant réservoir ; une zone sécable destinée à libérer une ouverture d'expulsion; et une zone de préhension comprenant un chemin de fuite du contenu, fermé en l'absence d'une pression de l'utilisateur afin de bloquer la sortie du contenu après ouverture du conditionnement.

Le document EP 0 150 751 décrit un emballage distributeur du type jetable comprenant un corps de récipient présentant des parties plates en ailettes latérales ainsi qu'un col sécable pour former une ouverture d'expulsion du contenu.

De tels conditionnements ne comprennent qu'une simple ouverture de distribution du contenu, étant dépourvus d'un véritable moyen de distribution et d'application du contenu et ne permettant pas une application de contenu précise quant à la taille, la forme, la limite, et faible quant à la dimension, la surface, la quantité, comme par exemple pour un vernis à ongle.

Le document WO 2008/025897 décrit un dispositif comprenant un réservoir délimité par une zone de solidarisation de deux ensembles et qui comporte un orifice de distribution, et un applicateur comprenant un organe de préhension et un organe de répartition composé d'une tige traversant l'orifice de distribution, dont la première extrémité est munie d'un embout de répartition du contenu et dont la seconde extrémité est incluse dans l'organe de préhension. L'un des ensembles est une coque comprenant une cavité ayant une collerette dont fait partie la zone de solidarisation et l'un des ensembles a une collerette comportant une gouttière. L'orifice de distribution est en communication avec le réservoir par un goulot formé par l'espace entre les deux ensembles après solidarisation, la gouttière formant au moins en partie une paroi du goulot. Le goulot comporte une structure sécable, de sorte que la rupture d'une zone de solidarisation réduite ouvre l'orifice de distribution. Le goulot a une structure étroite et relativement circulaire de sorte à former un organe d'essorage de l'applicateur. L'orifice de distribution est obturé par un élément d'obturation formé par la solidarisation d'une expansion de la collerette. L'organe de préhension de l'applicateur est formé par l'élément d'obturation. Un tel conditionnement est trop complexe et inadapté lorsqu'il n'y a pas lieu de prévoir un organe d'essorage.

Le document FR 2 837 174 décrit un conditionnement qui comprend un moyen de réception et d'expulsion comportant une cavité pourvue d'une sortie délimitée par deux parois, dont une au moins déformable, associées de façon fixe et étanche dans une région périphérique, apte à contenir du contenu, dont une certaine quantité peut être expulsée par la sortie lorsqu'elle est ouverte, par suite d'une pression d'origine extérieure sur la paroi déformable ; un moyen de manoeuvre responsif à une telle pression, apte à déformer la paroi déformable, sous la forme d'une pièce séparée supplémentaire constituant une coque elle aussi déformable ; un moyen de distribution et d'application, incluant une partie attenante à, et en communication avec, la sortie et un tampon d'application ; un moyen de fermeture/ouverture de la sortie, frangible, apte à fermer la sortie avant la première utilisation, et, après rupture, à ouvrir la sortie en vue de la première utilisation, constitué soit par un pointeau de percement soit par une soudure faible ; et un moyen de protection sous la forme d'un capot amovible monté sur la coque. Un tel conditionnement est totalement inadapté au domaine de l'invention comme précédemment exposé.

L'état de la technique est également illustré par les documents US 2010/001881 et EP 1 972 358, qui décrivent des dispositifs qui, comme le précédent, sont totalement inadaptés au domaine de l'invention.

Le document DE 3122237 décrit un dispositif comprenant, avec un axe, une paroi semi-rigide conformée, une paroi de fermeture et un applicateur ayant une partie de fixation et une partie d'application en prolongation mutuelle, tel que :
▪ la paroi semi-rigide conformée comporte une bordure périphérique plane et un creux localisé d'un côté du plan de la bordure et entouré par elle, ayant, en prolongation mutuelle et communication, une portion de réception de contenu, déformable moyennant une pression extérieure, une portion de fixation avec saillie intérieure et une portion de protection coopérant, respectivement, avec la partie de fixation et la partie d'application de l'applicateur,
▪ la paroi de fermeture couvre la paroi semi-rigide conformée, est fixée rigidement à la bordure, et ferme hermétiquement le creux en ménageant un réservoir de contenu, une cavité de fixation de la partie de fixation de l'applicateur, et une enceinte de protection de la partie d'application de l'applicateur qui est apte, lors de l'opération d'ouverture / dissociation, à être dissociée de cette partie d'application pour la dégager,
▪ un moyen sécable de la paroi semi-rigide conformée et de la paroi de fermeture est localisé transversalement entre la portion de fixation et la portion de réception, et est apte à être rompu pour l'opération d'ouverture / dissociation,
▪ un passage de transfert de contenu s'étend le long de l'axe entre, et en communication avec, le réservoir de contenu et la partie d'application de l'applicateur, de sorte que, pour l'utilisation du dispositif à l'état ouvert / dissocié, du contenu est transféré du réservoir de contenu à la partie d'application de l'applicateur suite à une pression extérieure sur la portion de réception de la paroi semi-rigide conformée.

La face externe de la partie de fixation de l'applicateur et la face intérieure en regard de la portion de fixation de la paroi semi-rigide conformée sont en contact étanche et assurent l'immobilisation de l'applicateur. Cette étanchéité est renforcée par la saillie prévue sur la face intérieure de la portion de fixation venant enserrer en pénétrant la partie de fixation de l'applicateur. Compte tenu de cette étanchéité, il est prévu que le passage de transfert de contenu est un canal traversant la partie de fixation de l'applicateur de part en part. Ainsi, le contenu traverse de part en part la partie de fixation de l'applicateur depuis le réservoir de contenu jusqu'à la partie d'application de l'applicateur qu'il atteint par l'intérieur. Cette disposition, inhérente à la structure du dispositif ne permet pas d'appliquer sur la surface d'application, de façon efficace, et comme avec un pinceau, un contenu sous forme de liquide, de gel, de crème, ou de pâte, tel que par exemple un produit cosmétique, un produit de soin corporel, un produit d'hygiène corporelle, un médicament, un produit d'entretien, un produit de décoration, un produit d'écriture, un produit de masquage, une peinture, une encre, une laque, un vernis, de façon précise quant à la taille, la forme, la limite, et faible quant à la taille, la surface, la quantité.

Le document DE 10 2009 029 concerne un dispositif différent mais qui présente une structure analogue avec étanchéité périphérique.

Le premier problème que résout l'invention est donc de s'affranchir d'une structure a étanchéité périphérique et passage traversant l'applicateur.

De plus, et s'agissant d'un conditionnement à usage unique, pour une dose limitée de contenu tel qu'une dose unique, il importe que le conditionnement soit de constitution simple avec le minimum de pièces et de fabrication également aisée et rapide, aisément machinable.
Tel est le second problème que résout l'invention.

Ci-après, un exposé de l'invention telle qu'elle est caractérisée.

Selon un aspect, l'invention a pour objet un dispositif pour contenir, distribuer, appliquer sur une surface d'application un contenu sous forme de liquide, gel, crème, pâte, à l'état fermé / associé avant utilisation, apte à être amené à l'état ouvert / dissocié pour son utilisation moyennant une opération d'ouverture / dissociation, comprenant, avec un axe, une paroi semi-rigide conformée, une paroi de fermeture et un applicateur ayant une partie de fixation et une partie d'application en prolongation mutuelle, tel que :
▪ la paroi semi-rigide conformée comporte une bordure périphérique plane et un creux localisé d'un côté du plan de la bordure et entouré par elle, ayant, en prolongation mutuelle et communication, une portion de réception de contenu, déformable moyennant une pression extérieure, une portion de fixation avec saillie intérieure et une portion de protection coopérant, respectivement, avec la partie de fixation et la partie d'application de l'applicateur,
▪ la paroi de fermeture couvre la paroi semi-rigide conformée, est fixée rigidement à la bordure, et ferme hermétiquement le creux en ménageant un réservoir de contenu, une cavité de fixation de la partie de fixation de l'applicateur, et une enceinte de protection de la partie d'application de l'applicateur qui est apte, lors de l'opération d'ouverture / dissociation, à être dissociée de cette partie d'application pour la dégager,
▪ un moyen sécable de la paroi semi-rigide conformée et de la paroi de fermeture est localisé transversalement entre la portion de fixation et la portion de protection, et est apte à être rompu pour l'opération d'ouverture / dissociation,
▪ un passage de transfert de contenu s'étend le long de l'axe entre, et en communication avec, le réservoir de contenu et la partie d'application de l'applicateur, de sorte que, pour l'utilisation du dispositif à l'état ouvert / dissocié, du contenu est transféré du réservoir de contenu à la partie d'application de l'applicateur suite à une pression extérieure sur la portion de réception de la paroi semi-rigide conformée.

Ce dispositif est tel que le passage de transfert de contenu est réalisé avec une structure à écartement radial assurant une absence d'étanchéité périphérique entre la face périphérique externe de la partie de fixation de l'applicateur et la face périphérique intérieure en regard de la portion de fixation de la paroi semi-rigide conformée, la structure à écartement radial s'étendant de façon communicante et continue, le long de l'axe, entre, d'un côté, le réservoir de contenu et, de l'autre côté, le moyen sécable et la partie d'application de l'applicateur, et que l'alimentation en contenu à appliquer de la partie d'application de l'applicateur est réalisée à partir de son enveloppe périphérique extérieure, alors que le contenu est apte à pouvoir traverser le passage de transfert et à être appliqué comme avec un pinceau.

Ainsi, selon une caractéristique, la partie de fixation de l'applicateur est agencée de sorte que du contenu ne peut la traverser le long de l'axe, en particulier cette partie de fixation est pleine ou dépourvue de canal traversant ou de pores. C'est ainsi que l'invention résout le problème posé par les distributeurs de l'état de la technique connu.

Selon une réalisation, le passage de transfert de contenu est réalisé avec une structure à écartement radial entre, en outre, la face périphérique externe de la partie de fixation de l'applicateur et la face en regard de la paroi de fermeture.

Selon une réalisation, le passage de transfert de contenu comprend essentiellement, en particulier est constitué par, la structure à écartement radial entre la face périphérique externe de la partie de fixation de l'applicateur et la face périphérique intérieure en regard de la portion de fixation de la paroi semi-rigide conformée.

Selon une réalisation, le passage de transfert de contenu comprend essentiellement, en particulier est constitué par, au moins un canal, et en particulier plusieurs canaux espacés angulairement, disposé axialement, limité par la face périphérique externe de la partie de fixation de l'applicateur et la face périphérique intérieure en regard de la portion de fixation de la paroi semi-rigide conformée, moyennant la structure à écartement radial, s'étendant de façon communicante et continue et débouchant d'un côté, dans le réservoir de contenu et, de l'autre côté, vers, en particulier de façon adjacente à, l'enveloppe périphérique extérieure de la partie d'application de l'applicateur située vers la partie de fixation de l'applicateur, en particulier sur la face extérieure même de la partie d'application de l'applicateur.

Selon une réalisation, le dispositif est avec plusieurs canaux disposés au moins sensiblement symétriquement par rapport à un plan médian du dispositif, orthogonal au plan de la bordure périphérique de la paroi semi-rigide conformée.

Selon une réalisation, le dispositif comprend au moins un canal agencé de sorte à être disposé en étant soit le plus écarté radialement soit le moins écarté radialement de la paroi de fermeture, et en particulier comprend au moins deux canaux agencés de sorte à être disposés l'un le plus écarté radialement l'autre le moins écarté radialement de la paroi de fermeture.

Selon une réalisation, un canal a, en section transversale, une forme aplatie s'étendant dans la direction périphérique de la face périphérique externe de la partie de fixation de l'applicateur et de la face périphérique intérieure en regard de la portion de fixation de la paroi semi-rigide conformée.

Selon une réalisation, un canal a une section élargie vers le réservoir de contenu et/ou vers l'enveloppe périphérique extérieure de la partie d'application de l'applicateur. En particulier, un canal a, vers l'enveloppe périphérique extérieure de la partie d'application de l'applicateur, une section élargie dans la direction périphérique de la face périphérique externe de la partie de fixation de l'applicateur et de la face périphérique intérieure en regard de la portion de fixation de la paroi semi-rigide conformée, de sorte à représenter au moins 20%, en particulier au moins 40%, plus spécialement au moins 60% et éventuellement la totalité ou la presque totalité de l'étendue périphérique de l'enveloppe périphérique extérieure de la partie d'application de l'applicateur ou de la face extérieure même de la partie d'application de l'applicateur.

Selon une réalisation :
- la portion de fixation de la paroi semi-rigide conformée a, au moins dans sa partie longitudinale médiane, en particulier dans toute ou sensiblement toute sa longueur, une section transversale intérieure de forme générale en U, U à branches s'écartant, V, ou de forme semi-circulaire,
- la cavité de fixation a, au moins dans sa partie longitudinale médiane, en particulier dans toute ou sensiblement toute sa longueur, une section transversale intérieure de forme générale en carré, rectangle, trapèze à grande base vers la paroi de fermeture, ou de forme de secteur semi-circulaire,
- la forme de la section transversale intérieure de la portion de fixation de la paroi semi-rigide conformée et la forme de la section transversale intérieure la de la cavité de fixation sont en correspondance avec la forme de la section transversale extérieure de la partie de fixation de l'applicateur pour former le au moins un canal du passage de transfert de contenu, avec la structure à écartement radial.

Selon une réalisation :
- la partie de fixation de l'applicateur a, au moins dans sa partie longitudinale médiane, en particulier dans toute ou sensiblement toute sa longueur, une section transversale extérieure en forme générale polygonale, ou incurvée, en particulier ellipsoïdale,
- la forme de la section transversale extérieure de la partie de fixation de l'applicateur étant en correspondance avec la forme de la section transversale intérieure de la portion de fixation de la paroi semi-rigide conformée et la forme de la section transversale intérieure de la cavité de fixation pour former le au moins un canal du passage de transfert de contenu, avec la structure à écartement radial.

Selon une réalisation, le dispositif combine la structure à écartement radial et une structure à contact ou quasi contact entre la face périphérique externe de la partie de fixation de l'applicateur et la face périphérique intérieure en regard de la portion de fixation de la paroi semi-rigide conformée, agencée pour laisser libre ou ne pas occulter le passage de transfert de contenu réalisé avec la structure à écartement radial, et remplir une fonction de positionnement radial fixe ou sensiblement fixe de la partie de fixation de l'applicateur dans la cavité de fixation.

Selon une réalisation, la structure à contact ou quasi contact entre la face périphérique externe de la partie de fixation de l'applicateur et la face périphérique intérieure en regard de la portion de fixation de la paroi semi-rigide conformée, est agencée avec une disposition ponctuelle, ou linéaire, en particulier dans la direction axiale et/ou dans la direction périphérique, ou surfacique, et est agencée en outre avec une disposition discontinue, en particulier une disposition répartie, et avec une localisation à la fois périphérique et axiale.

Selon une réalisation :
- la portion de fixation de la paroi semi-rigide conformée a, au moins dans sa partie longitudinale médiane, en particulier dans toute ou sensiblement toute sa longueur, une section transversale intérieure de forme générale en U, U à branches s'écartant, V, ou de forme semi-circulaire,
- la cavité de fixation a, au moins dans sa partie longitudinale médiane, en particulier dans toute ou sensiblement toute sa longueur, une section transversale intérieure de forme générale en carré, rectangle, trapèze à grande base vers la paroi de fermeture, ou de forme de secteur semi-circulaire,
- la partie de fixation de l'applicateur a, au moins dans sa partie longitudinale médiane, en particulier dans toute ou sensiblement toute sa longueur, une section transversale extérieure en forme générale polygonale, ou incurvée, en particulier ellipsoïdale,
- la forme de la section transversale extérieure de la partie de fixation de l'applicateur est en correspondance avec la forme de la section transversale intérieure de la portion de fixation de la paroi semi-rigide conformée et la forme de la section transversale intérieure de la cavité de fixation, de sorte à former la structure à contact ou quasi contact, assurant un contact ou un quasi-contact entre la face périphérique externe de la partie de fixation de l'applicateur et la face périphérique intérieure en regard de la portion de fixation de la paroi semi-rigide conformée, le contact ou un quasi-contact étant agencé pour ménager la structure à écartement radial.

Selon une réalisation, la portion de fixation de la paroi semi-rigide conformée comporte une structure à saillie intérieure agencée pour laisser libre ou ne pas occulter le passage de transfert de contenu réalisé avec la structure à écartement radial, et remplir une fonction de positionnement axial fixe ou sensiblement fixe de la partie de fixation de l'applicateur dans la cavité de fixation.

Selon une réalisation, la structure à saillie intérieure comprend essentiellement, en particulier est constitué par, au moins une saillie prévue sur la face périphérique intérieure de la portion de fixation de la paroi semi-rigide conformée, dirigée vers l'axe, et coopérant avec la face périphérique externe et/ou l'une au moins des faces transversales d'extrémité de la partie de fixation de l'applicateur de sorte à assurer le positionnement axial fixe ou sensiblement fixe de la partie de fixation de l'applicateur dans la cavité de fixation.

Selon une réalisation, une saillie de la structure à saillie intérieure est agencée avec une localisation transversale.

Selon une réalisation, une saillie de la structure à saillie intérieure est localisée sur une partie seulement de la face périphérique intérieure de la portion de fixation entre ses deux bords opposés. Selon une autre réalisation, une saillie de la structure à saillie intérieure est agencée de sorte que du contenu peut la traverser dans la direction de l'axe du dispositif, étant pourvue d'un passage traversant ou de pores ou étant formée de portions discontinues.

Selon une réalisation, la structure à saillie intérieure comprend deux saillies, l'une vers le réservoir de contenu et l'autre vers le moyen sécable, coopérant avec les deux faces transversales d'extrémité de la partie de fixation de l'applicateur placée entre les deux saillies.

Selon une réalisation, le moyen sécable comprend une ligne ou une aire frangible de plus faible résistance, localisée dans une région de plus petite largeur transversale du dispositif dans le plan de la bordure périphérique de la paroi semi-rigide conformée.

Selon une réalisation, le moyen sécable est choisi de type casser-tourner ou snap and twist.

Selon une réalisation, l'applicateur comporte une partie de fixation telle qu'une tige, de forme générale cylindrique, et une partie d'application telle qu'un pinceau, un tampon, une palette ou une tige, en particulier une partie d'application adaptée pour une application précise quant à la taille, la forme, la limite, faible quant à la dimension, la surface, la quantité.

Selon une réalisation, la paroi semi-rigide conformée est une paroi thermoformée et la paroi de fermeture est un opercule plan ou une seconde paroi thermoformée plane ou conformée en creux.

Selon un développement, à la paroi de fermeture est intégré ou rapporté de façon fixe un conteneur accessoire ou complémentaire pour un contenu accessoire ou complémentaire, en particulier tel qu'un sachet flexible.

Selon un autre développement, la paroi semi-rigide conformée comprend un second creux, distinct et espacé, fermé par une paroi de fermeture, de sorte à former un conteneur accessoire ou complémentaire pour un contenu accessoire ou complémentaire.

Selon un autre développement, il est prévu un ou plusieurs autres dispositifs analogues de sorte à former une plaque de dispositifs, en particulier côte à côte, en particulier séparés les uns des autres par des moyens sécables.

Selon une réalisation, le réservoir de contenu a une capacité pour un usage unique, en particulier comprise entre de l'ordre de 0,5 ml et de l'ordre de 10 ml.

Selon un aspect, le dispositif est dans la situation dans laquelle le réservoir de contenu est vide de contenu. Selon un autre aspect, le dispositif est dans la situation dans laquelle le réservoir de contenu est empli de contenu tel qu'un produit cosmétique, un produit de soin corporel, un produit d'hygiène corporelle, un médicament, un produit d'entretien, un produit de décoration, un produit d'écriture, un produit de masquage, une peinture, une encre, une laque, un vernis.

L'invention vise également un dispositif tel que décrit, une fois amené à l'état ouvert / dissocié pour son utilisation moyennant une opération d'ouverture / dissociation, par laquelle la partie sécable est rompue, qui comprend deux parties dissociées l'une de l'autre :
- une partie d'utilisation qui comprend le réservoir de contenu, la cavité de fixation de la partie de fixation de l'applicateur et l'applicateur dont la partie d'application, libre et saillant de la portion de fixation est découverte et peut être appliquée sur la surface d'application,
- une partie jetable qui comprend l'enceinte de protection dont la partie d'application de l'applicateur a été extraite et qui ne peut plus être remise en place sur la partie d'utilisation.

Selon une caractéristique, pour la mise en oeuvre du dispositif une fois amené à l'état ouvert / dissocié, le contenu est transféré du réservoir de contenu à la partie d'application de l'applicateur par l'extérieur moyennant, d'abord, une pression exercée brièvement sur la portion de réception de la paroi semi-rigide conformée pour amorcer le transfert, puis, le mouvement de la partie d'application de l'applicateur sur et au contact de la surface d'application.

On décrit maintenant brièvement les figures des dessins.

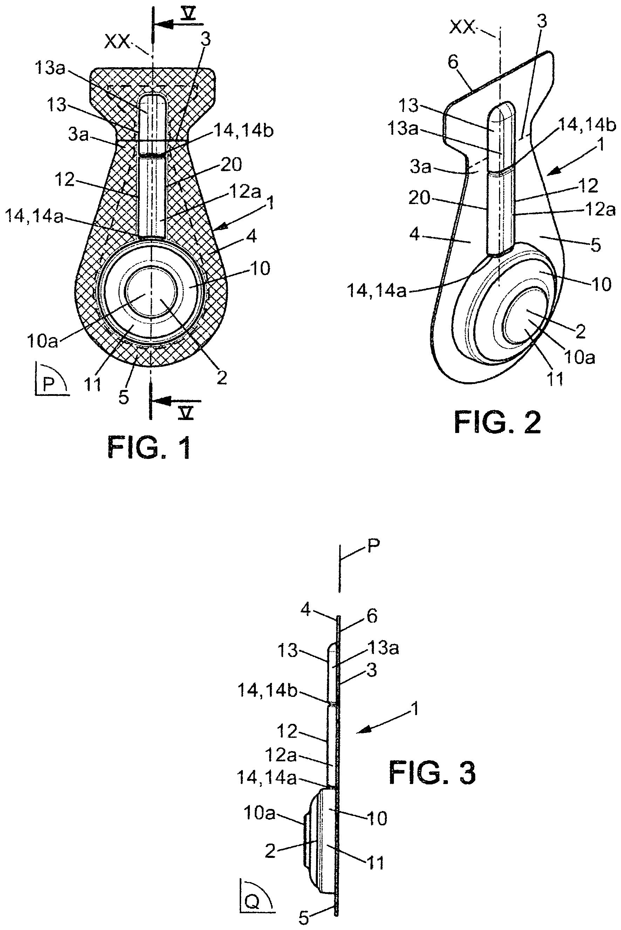

La figure 1 est une vue en élévation, côté paroi semi-rigide conformée, d'une dose d'un contenu conditionné dans une réalisation possible d'un dispositif pour contenir, distribuer, appliquer sur une surface d'application un tel contenu, à l'état fermé / associé avant utilisation, montrant plus particulièrement que la paroi semi-rigide conformée comporte une bordure périphérique, un moyen sécable et, en prolongation mutuelle, une portion de réception de contenu, une portion de fixation avec une structure à saillie intérieure comprenant deux saillies, l'une vers le réservoir de contenu et l'autre vers le moyen sécable, et une portion de protection.
La figure 2 est une vue en perspective du dispositif de la figure 1, côté paroi semi-rigide conformée.
La figure 3 est une vue de côté du dispositif de la figure 1, montrant plus particulièrement la paroi semi-rigide conformée et la paroi de fermeture, ici un opercule plan.
La figure 4 est une vue en élévation du dispositif de la figure 1 à l'état ouvert / dissocié, comprenant la figure 4A montrant la partie d'utilisation du dispositif comprenant le réservoir de contenu, la cavité de fixation de la partie de fixation de l'applicateur et la partie d'application de l'applicateur, libre, saillant de la portion de fixation, découverte et pouvant être appliquée sur une surface d'application, et la figure 4B montrant la partie jetable du dispositif comprenant l'enceinte de protection dont la partie d'application de l'applicateur a été extraite.

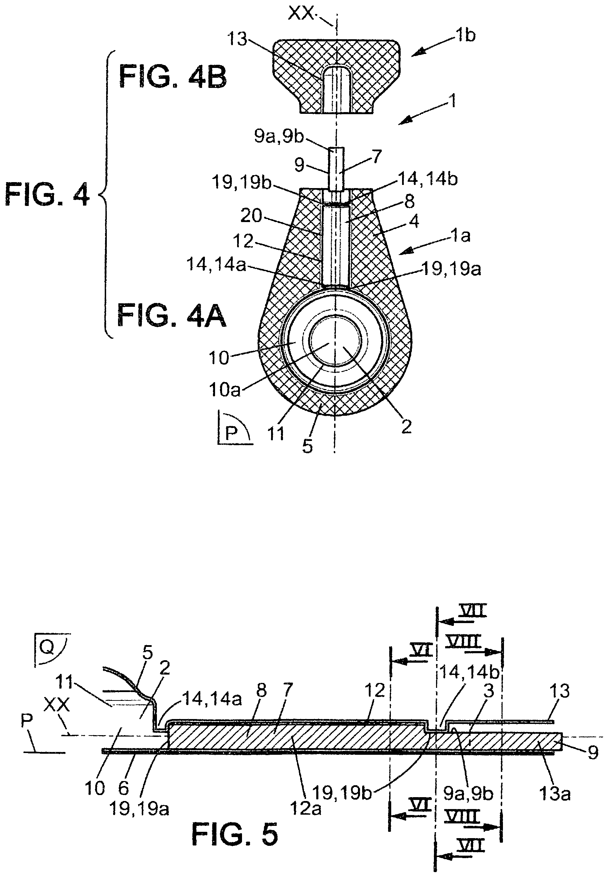

La figure 5 est une vue en coupe axiale, partielle, du dispositif, selon la ligne V-V de la figure 1, montrant plus particulièrement, en premier lieu, la paroi de fermeture, en deuxième lieu, pour la paroi semi-rigide conformée, une partie de la portion de réception de contenu, la portion de fixation avec une structure à saillie intérieure comprenant deux saillies, l'une vers le réservoir de contenu et l'autre vers le moyen sécable, coopérant avec les deux faces transversale d'extrémité de la partie de fixation de l'applicateur placée entre les deux saillies, et la portion de protection, en troisième lieu, la partie de fixation de l'applicateur agencée de sorte que du contenu ne peut la traverser, en quatrième lieu, l'applicateur avec sa partie de fixation dans la portion de fixation et sa partie d'application dans la portion de protection, et enfin le passage de contenu vers l'enveloppe périphérique extérieure et plus particulièrement sur la face extérieure même de la partie d'application de l'applicateur.

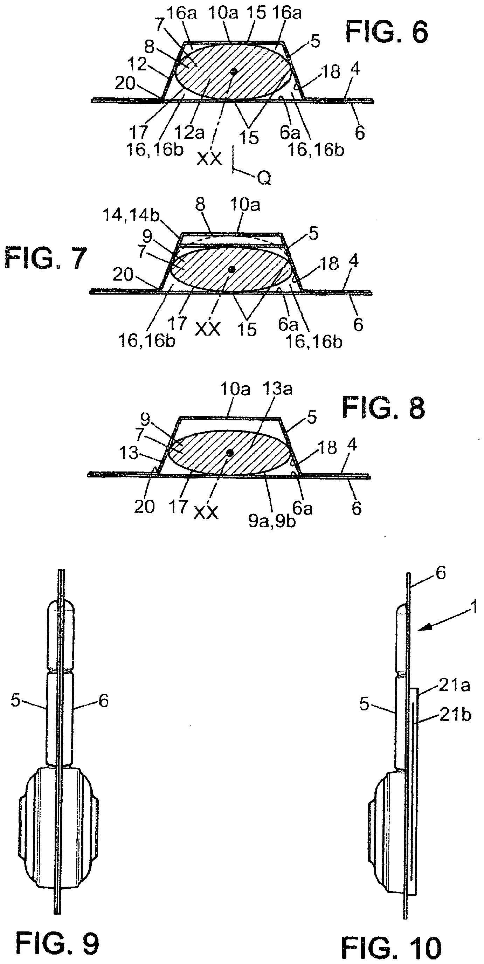

La figure 6 est une vue en coupe transversale du dispositif, selon la ligne VI-VI de la figure 5, montrant plus particulièrement, en premier lieu, que le passage de transfert de contenu est réalisé avec une structure à écartement radial assurant une absence d'étanchéité périphérique entre la face périphérique externe de la partie de fixation de l'applicateur et la face périphérique intérieure de la portion de fixation de la paroi semi-rigide conformée, en deuxième lieu, que la partie de fixation de l'applicateur est agencée de sorte que du contenu ne peut la traverser, en troisième lieu, que le passage de transfert comprend ici quatre canaux symétriques par rapport au plan médian du dispositif, à savoir deux canaux écartés radialement et deux canaux proches radialement de la paroi de fermeture, ces canaux ayant en section transversale une forme aplatie dans la direction périphérique de la face périphérique externe de la partie de fixation et de la face périphérique intérieure de la portion de fixation, et en quatrième lieu que la structure à écartement radial est combinée à une structure à contact ou quasi contact entre la face périphérique externe de la partie de fixation et la face périphérique intérieure de la portion de fixation.
La figure 7 est une vue en coupe transversale analogue à la figure 6, selon la ligne VII-VII de la figure 5, montrant plus particulièrement, la saillie de la structure à saillie intérieure située vers le moyen sécable, coopérant avec la face transversale d'extrémité correspondante de la partie de fixation de l'applicateur.
La figure 8 est une vue en coupe transversale analogue à la figure 6, selon la ligne VIII-VIII de la figure 5, montrant plus particulièrement, que le passage de contenu distribue le contenu vers l'enveloppe périphérique extérieure et plus particulièrement sur la face extérieure même de la partie d'application de l'applicateur.
La figure 9 est une vue de côté du dispositif avec une paroi de fermeture qui est ici une seconde paroi thermoformée conformée en creux, symétrique de la première.
La figure 10 est une vue de côté du dispositif de la figure 1, dans le cas où à la paroi de fermeture duquel est intégré ou rapporté de façon fixe un conteneur accessoire ou complémentaire pour un contenu accessoire ou complémentaire, tel qu'un sachet plat.
La figure 11 est une vue en élévation, côté paroi semi-rigide conformée, du dispositif, dans lequel la paroi semi-rigide conformée comprend deux seconds creux, distincts et espacés, fermés, de sorte à former des conteneurs accessoires ou complémentaires.
La figure 12 est une vue en élévation, côté paroi semi-rigide conformée, montrant un dispositif agencé avec un autre dispositif analogues de sorte à former une plaque de dispositifs, côte à côte, séparés par des moyens sécables.

Ci-après un exposé détaillé assorti d'exemples et de références aux dessins de plusieurs modes de réalisation d'un dispositif 1 pour contenir, distribuer, appliquer, notamment appliquer comme avec un pinceau, sur une surface d'application, un contenu sous forme de liquide, gel, crème, pâte.

Dans le contexte de l'invention, le dispositif 1 est tout spécialement adapté pour recevoir une dose du contenu en quantité pouvant être appropriée pour un usage unique, en particulier une quantité comprise entre 0,5 ml et 10 ml.

Ce contenu est par exemple un produit cosmétique, un produit de soin corporel, un produit d'hygiène corporelle, un médicament, un produit d'entretien, un produit de décoration, un produit d'écriture, un produit de masquage, une peinture, une encre, une laque, un vernis. Cette liste n'est pas limitative.

Ainsi, la surface d'application peut être celle de la peau, des ongles, des lèvres, d'un produit à entretenir, à décorer, sur lequel l'on veut écrire, sur lequel l'on veut faire un masquage, sur lequel l'on veut éppliquer une peinture, une laque, un vernis. Cette liste n'est pas limitative.

L'invention vise aussi bien la situation où le réservoir de contenu 2 du dispositif 1 est vide de contenu que la situation où le réservoir 2 est empli de contenu.

Avant utilisation, le dispositif 1 est à l'état fermé / associé (voir tout particulièrement les figures 1 à 3, 5 à 8). Il peut être amené à l'état ouvert / dissocié (voir la figure 4) pour son utilisation moyennant une opération d'ouverture / dissociation, le dispositif 1 comprenant un moyen sécable 3 sous la forme d'une ligne ou d'une aire frangible de plus faible résistance, en particulier de type casser-tourner, connu sous l'expression « snap and twist ».

Le dispositif 1 est tel que, à l'état ouvert / dissocié, il comporte une partie d'utilisation 1a et une partie jetable 1b qui ne peut plus être remise en place sur la partie d'utilisation 1a.

Dans le contexte de l'invention, le dispositif 1 est tout spécialement adapté pour une application de contenu précise quant à la taille, la forme, la limite, et faible quant à la dimension, la surface, la quantité.

De plus, le dispositif 1 est de type pouvant être portatif et nomade, léger et de petite taille - telle que par exemple de l'ordre de quelques centimètres pour sa plus grande longueur -, pouvant être rangés dans une poche, un sac, un vide-poche, un tiroir.

Le dispositif 1 comporte un axe XX et présente un plan d'extension P qui est celui d'une bordure périphérique plane 4 d'une paroi semi-rigide conformée 5. Le plan d'extension P est parallèle tout particulièrement au plan des figures 1 et 4. Le dispositif 1 présente un plan médian Q de symétrie globale, orthogonal au plan P et passant par l'axe XX. Le plan médian Q est parallèle tout particulièrement au plan des figures 3 et 5.

Le terme «axial », le terme « radial » et l'expression « espacé angulairement » se comprennent dans leur acception habituelle, ici en référence à l'axe XX. Le terme « transversal » se réfère à ce qui est perpendiculaire ou orthogonal à l'axe XX. Le terme « périphérique » vise ce qui est situé globalement sur un pourtour externe entourant l'axe XX.

Le dispositif 1 comprend trois pièces, à savoir la paroi semi-rigide conformée 5, ici une paroi thermoformée, une paroi de fermeture 6 et un applicateur 7. L'applicateur 7 est formé d'une partie de fixation 8, telle qu'une tige, pouvant être de forme générale cylindrique, et d'une partie d'application 9 en prolongation axiale mutuelle, telle qu'un pinceau, un tampon, une palette ou une tige, et en particulier une partie d'application adaptée pour une application précise quant à la taille, la forme, la limite, faible quant à la dimension, la surface, la quantité. L'applicateur 7 est en une pièce unique ou en plusieurs, notamment deux, pièces assemblées, par exemple la partie de fixation 8 et la partie d'application 9.

La paroi semi-rigide conformée 5 comporte la bordure périphérique 4 et un creux 10.

Le creux 10 est localisé d'un côté du plan P et entouré par la bordure 4.

Le creux 10 comporte, en prolongation axiale mutuelle et en communication, une portion de réception 11, une portion de fixation 12 et une portion de protection 13.

La paroi de fermeture 6 couvre la paroi semi-rigide conformée 5, est fixée rigidement à la bordure 4, et ferme hermétiquement le creux 10.

Dans la réalisation des figures 1 à 8 et 10 à 12, la paroi de fermeture 6 est un opercule tendu, plan ou sensiblement plan. Dans la réalisation de la figure 9, la paroi de fermeture 6 est une seconde paroi thermoformée plane ou conformée en creux, notamment plus ou moins comme la paroi semi-rigide conformée 5, en particulier identique à celle-ci, et disposée symétriquement par rapport au plan d'extension P.

La paroi de fermeture 6 ménage avec la portion de réception 11 du creux 10 un réservoir de contenu 2. Elle ménage avec la portion de fixation 12 du creux 10 une cavité de fixation 12a. Elle ménage avec la portion de protection 13 du creux 10 une enceinte de protection 13a.

La portion de réception 11, respectivement le réservoir de contenu 2, ici avec une forme proche de celle d'un secteur sphérique, est apte à recevoir la dose de contenu.

La portion de réception 11 est déformable moyennant une pression extérieure orthogonale au plan P, comme celle pouvant être exercée par l'utilisateur avec les doigts.

La portion de fixation 12, allongée, rectiligne, est pourvue d'une structure à saillie intérieure 14 qui remplit une fonction de positionnement axial fixe ou sensiblement fixe de la partie de fixation 8 de l'applicateur 7 dans la cavité de fixation 12a, et donc plus généralement de l'applicateur 7 par rapport à la paroi semi-rigide conformée 5.

La portion de fixation 12 participe à une structure à contact ou quasi contact 15 qui remplit une fonction de positionnement radial fixe ou sensiblement fixe de la partie de fixation 8 de l'applicateur 7 dans la cavité de fixation 12a, et donc plus généralement de l'applicateur 7 par rapport à la paroi semi-rigide conformée 5.

La portion de fixation 12, respectivement la cavité de fixation 12a, d'une part, la portion de protection 13, respectivement l'enceinte de protection 13a, coopèrent, respectivement, avec la partie de fixation 8 et la partie d'application 9 de l'applicateur 7. L'enceinte de protection 13a est apte, lors de l'opération d'ouverture / dissociation, à être dissociée de la partie d'application 9 de l'applicateur 7 pour la dégager, comme illustré par la figure 4.

Le moyen sécable 3 concerne la paroi semi-rigide conformée 5 et la paroi de fermeture 6.
Le moyen sécable 3 est localisé transversalement entre la portion de fixation 12 et la portion de protection 13, notamment est localisé dans une région 3a de plus petite largeur transversale du dispositif 1 dans le plan d'extension P.

Le dispositif 1 comporte également un passage de transfert de contenu 16 réalisé avec une structure à écartement radial. Ce passage 16 est permanent et venu de fabrication, d'origine.

Le passage 16 s'étend le long de l'axe XX entre, et en communication avec, le réservoir de contenu 2 et la partie d'application 9 de l'applicateur 7.

Ainsi, pour l'utilisation du dispositif 1 à l'état ouvert / dissocié, du contenu est transféré du réservoir de contenu 2 à la partie d'application 9 de l'applicateur 7, par l'extérieur de cette dernière, suite à une pression extérieure sur la portion de réception 11 de la paroi semi-rigide conformée 5.

La structure à écartement radial assure une absence d'étanchéité périphérique entre la face périphérique externe 17 de la partie de fixation 8 de l'applicateur 7 et la face périphérique intérieure 18, en regard, de la portion de fixation 12 de la paroi semi-rigide conformée 5.

Selon une réalisation, le passage 16 comprend essentiellement, en particulier est constitué par, la structure à écartement radial entre la face périphérique externe 17 et la face périphérique intérieure 18.

Selon une possibilité, le passage 16 est réalisé avec une structure à écartement radial entre, en outre, la face périphérique externe 17 de la partie de fixation 8 de l'applicateur 7 et la face intérieure 6a, en regard, de la paroi de fermeture 6 sous forme d'opercule.

La structure à écartement radial s'étend de façon communicante et continue, le long de l'axe XX, entre, d'un côté, le réservoir de contenu 2 et, de l'autre côté, le moyen sécable 13 et la partie d'application 9 de l'applicateur 7.

La structure est ainsi agencée pour que l'alimentation en contenu à appliquer, de la partie d'application 9 de l'applicateur 7, est réalisée à partir de son enveloppe périphérique extérieure 9a, de forme générale cylindrique, par exemple.

Le contenu est apte à pouvoir traverser le passage de transfert 16.

Inversement, le dispositif 1 est tel que la partie de fixation 8 de l'applicateur 7 est agencée de sorte que le contenu ne peut la traverser le long de l'axe XX, en particulier parce que cette partie de fixation 8 est pleine ou dépourvue de canal traversant ou de pores.

Ainsi, comme représenté plus particulièrement sur les figures 6 et 7, le passage 16 comprend quatre canaux, à savoir deux canaux 16a et deux canaux 16b, espacés angulairement, disposés axialement, limités par la face périphérique externe 17 de la partie de fixation 8 de l'applicateur 7 et la face périphérique intérieure 18 en regard de la portion de fixation 12 de la paroi semi-rigide conformée 5, moyennant la structure à écartement radial. Ces canaux 16a, 16b s'étendant de façon communicante et continue et débouchent d'un côté, dans le réservoir de contenu 2 et, de l'autre côté, vers, et en particulier de façon adjacente à, l'enveloppe périphérique extérieure 9a de la partie d'application 9 de l'applicateur 7, située vers la partie de fixation 8 de l'applicateur 7, et plus particulièrement encore sur la face périphérique extérieure 9b même de la partie d'application 9 de l'applicateur 8.

Les deux canaux 16a et les deux canaux 16b, respectivement, sont disposés symétriquement par rapport au plan médian Q. Les deux canaux 16a sont agencés de sorte à être disposé en étant le plus écarté radialement de la paroi de fermeture 6, se trouvant vers le fond 10a du creux 10 opposé à la bordure 4. Les deux canaux 16b sont agencés de sorte à être disposé en étant le plus proche radialement de la paroi de fermeture 6, se trouvant vers la bordure 4.

Cette réalisation n'est pas exclusive d'autres dans lesquelles le passage 16 comprend essentiellement, en particulier est constitué par, au moins un canal, et en particulier plusieurs canaux espacés angulairement.

Comme représenté plus particulièrement sur les figures 6 et 7, un canal 16a, 16b a, en section transversale, une forme aplatie s'étendant dans la direction périphérique de la face périphérique externe 17 de la partie de fixation 9 de l'applicateur 7 et de la face périphérique intérieure 18 en regard de la portion de fixation 12 de la paroi semi-rigide conformée 5.

Selon une possibilité, il est prévu qu'un canal 16a, 16b, présente une section élargie vers le réservoir de contenu 2, de sorte à favoriser l'entrée du contenu dans le canal 16a, 16b réservoir de contenu 2.

Selon une autre possibilité, supplémentaire ou alternative, il est prévu qu'un canal 16a, 16b, présente une section élargie vers l'enveloppe périphérique extérieure 9a de la partie d'application 9 de l'applicateur 7, de sorte à favoriser la répartition du contenu sur la partie d'application 9. En particulier, cette section élargie représente au moins 20%, en particulier au moins 40%, plus spécialement au moins 60% et éventuellement la totalité ou la presque totalité de l'étendue périphérique de l'enveloppe périphérique extérieure 9a de la partie d'application de l'applicateur ou de la face extérieure 9b de la partie d'application 9 de l'applicateur 7.

La structure à saillie intérieure 14 et la structure à contact ou quasi contact 15 sont agencées pour laisser libre ou ne pas occulter le passage 16, ici les canaux 16a, 16b.

La structure à contact ou quasi contact 15, outre de remplir la fonction de positionnement radial fixe ou sensiblement fixe de la partie de fixation 8 de l'applicateur 7 dans la cavité de fixation 12a, participe à la formation de la structure à écartement radial et à la réalisattion du passage 16 et des canaux 16a et 16b.

Dans la réalisation représentée sur la figure 6, en premier lieu, la portion de fixation 12 de la paroi semi-rigide conformée 5 a une section transversale intérieure de forme générale en U à branches s'écartant vers l'ouverture opposée au fond 10a, la cavité de fixation 12a ayant ainsi une section transversale intérieure de forme générale en trapèze à grande base vers la paroi de fermeture 6 et à petite base vers le fond 10a.

En deuxième lieu, la partie de fixation 8 de l'applicateur 7 a une section transversale extérieure en forme générale ellipsoïdale,

En troisième lieu, la forme de la section transversale intérieure de la portion de fixation 12 de la paroi semi-rigide conformée 5 et la forme de la section transversale intérieure de la cavité de fixation 12a, d'une part, et la forme de la section transversale extérieure de la partie de fixation 8 de l'applicateur 7, d'autre part, sont en correspondance pour former les canaux 16a et 16b, avec la structure à écartement radial et pour former former la structure à contact ou quasi contact 15 laquelle est agencée pour ménager la structure à écartement radial, comme exposé précédemment.

Cette réalisation n'est pas exclusive d'autres, dès lors que les fonctions précédemment décrites sont remplies.

Ainsi, de façon plus générale, la portion de fixation 12 de la paroi semi-rigide conformée 5 a, au moins dans sa partie longitudinale médiane, en particulier dans toute ou sensiblement toute sa longueur, une section transversale intérieure de forme générale pouvant aussi être en U, en V, ou de forme semi-circulaire. Et la cavité de fixation 12a a, au moins dans sa partie longitudinale médiane, en particulier dans toute ou sensiblement toute sa longueur, une section transversale intérieure de forme générale pouvant aussi être en carré, rectangle, ou de forme de secteur semi-circulaire. Et la partie de fixation 8 de l'applicateur 7 a, au moins dans sa partie longitudinale médiane, en particulier dans toute ou sensiblement toute sa longueur, une section transversale extérieure pouvant aussi être en forme générale polygonale, ou incurvée.

Dans la réalisation représentée sur la figure 6, la structure à contact ou quasi contact 15 est agencée avec une disposition quadruplement sensiblement linéaire (ou surfaciquement peu étendue dans la direction périphérique) dans la direction axiale, ce qui conduit à une disposition répartie en quatre endroits.

Cette réalisation n'est pas exclusive d'autres dans lesquelles la structure à contact ou quasi contact 15 est agencée avec une disposition ponctuelle dans la direction axiale et/ou dans la direction périphérique, ou surfacique, ou bien est agencée, en outre, avec une disposition discontinue.

De façon générale, la structure à saillie intérieure 14 comprend essentiellement, en particulier est constitué par, au moins une saillie intérieure, agencée avec une localisation transversale, prévue sur la face périphérique intérieure 18 de la portion de fixation 12 de la paroi semi-rigide conformée 5, dirigée vers l'axe XX, et coopérant avec la face périphérique externe 17 et/ou l'une au moins des faces transversales d'extrémité 19 de la partie de fixation 8 de l'applicateur 7.

De façon générale et selon les réalisations, une saillie de la structure à saillie intérieure 14 est localisée sur une partie seulement de la face périphérique intérieure 18 de la portion de fixation 12 entre ses deux bords opposés 20, ou bien elle est agencée de sorte que du contenu peut la traverser dans la direction de l'axe XX, étant pourvue d'un passage traversant ou de pores ou étant formée de portions discontinues.

Dans la réalisation particulière représentée plus spécialement sur les figures 1 à 5 et 8, la structure à saillie intérieure 14 comprend deux saillies, l'une 14a vers le réservoir de contenu 2 et l'autre 14b vers le moyen sécable 3. Ces deux saillies 14a et 14b coopèrent avec les deux faces transversales d'extrémité 19a, 19b, respectivement, de la partie de fixation 8 de l'applicateur 7, laquelle partie de fixation 8 est placée entre les deux saillies 14a et 14b de sorte à être immobilisée axialement.

Selon un développement illustré par la figure 10, à la paroi de fermeture 6 est intégré ou rapporté de façon fixe un (ou plusieurs) conteneurs accessoires ou complémentaires 21a pour un (ou plusieurs) contenus accessoires ou complémentaires 21b. Un tel conteneur accessoire ou complémentaire 21a peut être, en particulier, un sachet flexible collé ou soudé sur la face extérieure de la paroi de fermeture 6.

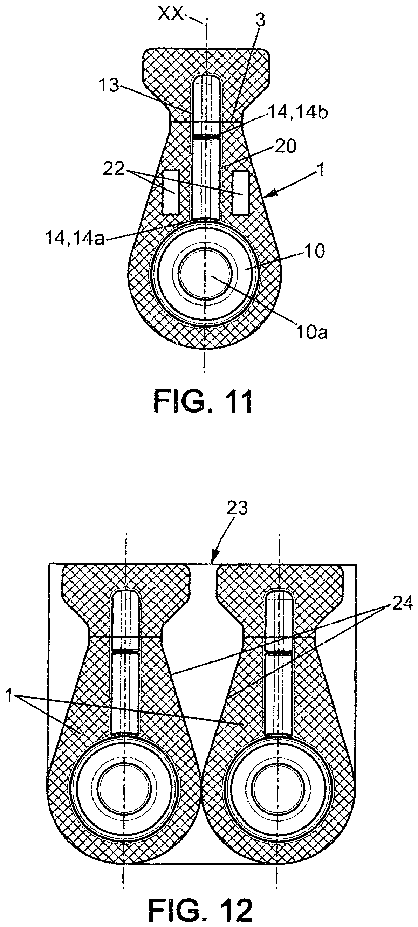

Selon un autre développement illustré par la figure 11, la paroi semi-rigide conformée 5 comprend un (ou plusieurs) seconds creux 22, distincts et espacés du creux 10, fermés par une paroi de fermeture qui peut être la paroi de fermeture 6, de sorte à former un (ou plusieurs) conteneurs accessoires ou complémentaires pour un (ou plusieurs) contenus accessoiress ou complémentaire.

Selon un autre développement illustré par la figure 12, il est prévu un (ou plusieurs) autres dispositifs 1, analogues au dispositif 1 précédemment décrit, de sorte à former une plaque 23 de dispositifs, en particulier côte à côte, en particulier séparés les uns des autres par des moyens sécables 24.

Un dispositif 1 tel qu'il a été décrit peut être amené à l'état ouvert / dissocié pour son utilisation moyennant une opération d'ouverture / dissociation, par laquelle la partie sécable 3 est rompue (figure 4). Il comprend alors deux parties dissociées l'une de l'autre, à savoir la partie d'utilisation 1a et la partie jetable 1 b.

Selon une caractéristique, pour la mise en oeuvre du dispositif 1 une fois amené à l'état ouvert / dissocié, le contenu est transféré du réservoir de contenu 2 à la partie d'application 9 de l'applicateur7 moyennant, d'abord, une pression exercée brièvement sur la portion de réception 11 de la paroi semi-rigide conformée 5 pour amorcer le transfert, puis, le mouvement de la partie d'application 9 de l'applicateur 7 sur et au contact de la surface d'application.

## Revendications

1. Dispositif (1) pour contenir, distribuer, appliquer sur une surface d'application un contenu sous forme de liquide, gel, crème, pâte, à l'état fermé / associé avant utilisation, apte à être amené à l'état-ouvert / dissocié,pour son utilisation moyennant une opération d'ouverture / dissociation, comprenant, avec un axe (XX), une paroi semi-rigide conformée (5), une paroi de fermeture (6) et un applicateur (7) ayant une partie de fixation (8) et une partie d'application (9) en prolongation mutuelle, tel que :
▪ la paroi semi-rigide conformée (5) comporte une bordure (4) périphérique plane et un creux (10) localisé d'un côté du plan (P) de la bordure (4) et entouré par elle, ayant, en prolongation mutuelle et communication, une portion de réception (11) de contenu, déformable moyennant une pression extérieure, une portion de fixation (12) avec saillie intérieure et une portion de protection (13) coopérant, respectivement, avec la partie de fixation (8) et la partie d'application (9) de l'applicateur (7),
▪ la paroi de fermeture (6) couvre la paroi semi-rigide conformée (5), est fixée rigidement à la bordure (4), et ferme hermétiquement le creux (10) en ménageant un réservoir (2) de contenu, une cavité de fixation (12a) de la partie de fixation (8) de l'applicateur (7), et une enceinte de protection (13a) de la partie d'application (9) de l'applicateur (7) qui est apte, lors de l'opération d'ouverture / dissociation, à être dissociée de cette partie d'application (9) pour la dégager,
▪ un moyen sécable (3) de la paroi semi-rigide conformée (5) et de la paroi de fermeture (6) est localisé transversalement entre la portion de fixation (12) et la portion de protection (13), et est apte à être rompu pour l'opération d'ouverture / dissociation,
▪ un passage de transfert (16) de contenu s'étend le long de l'axe (XX) entre, et en communication avec, le réservoir (2) de contenu et la partie d'application (9) de l'applicateur (7), de sorte que, pour l'utilisation du dispositif (1) à l'état ouvert / dissocié, du contenu est transféré du réservoir (2) de contenu à la partie d'application (9) de l'applicateur (7) suite à une pression extérieure sur la portion de réception (11) de la paroi semi-rigide conformée (5),
**caractérisé en ce que** le passage de transfert (16) de contenu est réalisé avec une structure à écartement radial assurant une absence d'étanchéité périphérique entre la face périphérique externe (17) de la partie de fixation (8) de l'applicateur (7) et la face périphérique intérieure (18) en regard de la portion de fixation (12) de la paroi semi-rigide conformée (5), la structure à écartement radial s'étendant de façon communicante et continue, le long de l'axe (XX), entre, d'un côté, le réservoir (2) de contenu et, de l'autre côté, le moyen sécable (3) et la partie d'application (9) de l'applicateur (7), et **en ce que** l'alimentation en contenu à appliquer de la partie d'application (9) de l'applicateur (7) est réalisée à partir de son enveloppe périphérique extérieure (9a), alors que le contenu est apte à pouvoir traverser le passage de transfert (16) et à être appliqué comme avec un pinceau.

2. Dispositif (1) selon la revendication 1, dans lequel la partie de fixation (8) de l'applicateur (7) est agencée de sorte que du contenu ne peut la traverser le long de l'axe (XX), en particulier dans lequel cette partie de fixation (8) est pleine ou dépourvue de canal traversant ou de pores.

3. Dispositif (1) selon l'une quelconque des revendications 1 et 2, dans lequel le passage de transfert (16) de contenu est réalisé avec une structure à écartement radial entre, en outre, la face périphérique externe (17) de la partie de fixation (8) de l'applicateur (7) et la face intérieure (6a) en regard de la paroi de fermeture (6).

4. Dispositif (1) selon l'une quelconque des revendications 1 à 3, dans lequel le passage de transfert (16) de contenu comprend essentiellement, en particulier est constitué par, au moins un canal, et en particulier plusieurs canaux (16a, 16b) espacés angulairement, disposé axialement, limité par la face périphérique externe (17) de la partie de fixation (8) de l'applicateur (7) et la face périphérique intérieure (18) en regard de la portion de fixation (12) de la paroi semi-rigide conformée (5), moyennant la structure à écartement radial, s'étendant de façon communicante et continue et débouchant d'un côté, dans le réservoir (2) de contenu et, de l'autre côté, vers, en particulier de façon adjacente à, l'enveloppe périphérique extérieure (9a) de la partie d'application (9) de l'applicateur (7) située vers la partie de fixation (8) de l'applicateur (7), en particulier sur la face extérieure (9b) même de la partie d'application (9) de l'applicateur (7), et optionnellement
comprenant plusieurs canaux (16a, 16b) disposés au moins sensiblement symétriquement par rapport à un plan médian (Q) du dispositif (1), orthogonal au plan (P) de la bordure (4) périphérique de la paroi semi-rigide conformée (5), et/ou comprenant au moins un canal agencé de sorte à être disposé en étant soit le plus écarté radialement soit le moins écarté radialement de la paroi de fermeture (6), et en particulier comprenant au moins deux canaux agencés de sorte à être disposés l'un le plus écarté radialement l'autre le moins écarté radialement de la paroi de fermeture (6).

5. Dispositif (1) selon la revendication 4, dans lequel un canal (16a, 16b) a, en section transversale, une forme aplatie s'étendant dans la direction périphérique de la face périphérique externe (17) de la partie de fixation (8) de l'applicateur (7) et de la face périphérique intérieure (18) en regard de la portion de fixation (12) de la paroi semi-rigide conformée (5), et optionnellement dans lequel
un canal (16a, 16b) a, vers l'enveloppe périphérique extérieure (9a) de la partie d'application (9) de l'applicateur (7), une section élargie dans la direction périphérique de la face périphérique externe (17) de la partie de fixation (8) de l'applicateur (7) et de la face périphérique intérieure (18) en regard de la portion de fixation (12) de la paroi semi-rigide conformée (5), de sorte à représenter au moins 20%, en particulier au moins 40%, plus spécialement au moins 60% et éventuellement la totalité ou la presque totalité de l'étendue périphérique de l'enveloppe périphérique extérieure (9a) de la partie d'application (9) de l'applicateur (7) ou de la face extérieure même de la partie d'application (9) de l'applicateur (7).

6. Dispositif (1) selon l'une quelconque des revendications 4 ou 5, dans lequel :
- la portion de fixation (12) de la paroi semi-rigide conformée (5) a, au moins dans sa partie longitudinale médiane, en particulier dans toute ou sensiblement toute sa longueur, une section transversale intérieure de forme générale en U, U à branches s'écartant, V, ou de forme semicirculaire,
- la cavité de fixation (12a) a, au moins dans sa partie longitudinale médiane, en particulier dans toute ou sensiblement toute sa longueur, une section transversale intérieure de forme générale en carré, rectangle, trapèze à grande base vers la paroi de fermeture (6), ou de forme de secteur semi-circulaire,
- la forme de la section transversale intérieure de la portion de fixation (12) de la paroi semi-rigide conformée (5) et la forme de la section transversale intérieure la de la cavité de fixation (12a) étant en correspondance avec la forme de la section transversale extérieure de la partie de fixation (8) de l'applicateur (7) pour former le au moins un canal du passage de transfert (16) de contenu, avec la structure à écartement radial,
et optionnellement dans lequel
- la partie de fixation (8) de l'applicateur (7) a, au moins dans sa partie longitudinale médiane, en particulier dans toute ou sensiblement toute sa longueur, une section transversale extérieure en forme générale polygonale, ou incurvée, en particulier ellipsoïdale,
- la forme de la section transversale extérieure de la partie de fixation (8) de l'applicateur (7) étant en correspondance avec la forme de la section transversale intérieure de la portion de fixation (12) de la paroi semi-rigide conformée (5) et la forme de la section transversale intérieure la de la cavité de fixation (12a) pour former le au moins un canal du passage de transfert (16) de contenu, avec la structure à écartement radial.

7. Dispositif (1) selon l'une quelconque des revendications 1 à 6, qui combine la structure à écartement radial et une structure à contact ou quasi contact (15) entre la face périphérique externe (17) de la partie de fixation (8) de l'applicateur (7) et la face périphérique intérieure (18) en regard de la portion de fixation (12) de la paroi semi-rigide conformée (5), agencée pour laisser libre ou ne pas occulter le passage de transfert (16) de contenu réalisé avec la structure à écartement radial, et remplir une fonction de positionnement radial fixe ou sensiblement fixe de la partie de fixation (8) de l'applicateur (7) dans la cavité de fixation (12a).

8. Dispositif (1) selon l'une quelconque des revendications 1 à 7, dans lequel :
- la portion de fixation (12) de la paroi semi-rigide conformée (5) a, au moins dans sa partie longitudinale médiane, en particulier dans toute ou sensiblement toute sa longueur, une section transversale intérieure de forme générale en U, U à branches s'écartant, V, ou de forme semi-circulaire,
- la cavité de fixation (12a) a, au moins dans sa partie longitudinale médiane, en particulier dans toute ou sensiblement toute sa longueur, une section transversale intérieure de forme générale en carré, rectangle, trapèze à grande base vers la paroi de fermeture (6), ou de forme de secteur semi-circulaire,
- la partie de fixation (8) de l'applicateur (7) a, au moins dans sa partie longitudinale médiane, en particulier dans toute ou sensiblement toute sa longueur, une section transversale extérieure en forme générale polygonale, ou incurvée, en particulier ellipsoïdale,
- la forme de la section transversale extérieure de la partie de fixation (8) de l'applicateur (7) étant en correspondance avec la forme de la section transversale intérieure de la portion de fixation (12) de la paroi semi-rigide conformée (5) et la forme de la section transversale intérieure la de la cavité de fixation (12a) de sorte à former la structure à contact ou quasi contact (15), assurant un contact ou un quasi-contact entre la face périphérique externe (17) de la partie de fixation (8) de l'applicateur (7) et la face périphérique intérieure (18) en regard de la portion de fixation (12) de la paroi semi-rigide conformée (5), le contact ou un quasi-contact étant agencé pour ménager la structure à écartement radial.

9. Dispositif (1) selon l'une quelconque des revendications 1 à 8, dans lequel la portion de fixation (12) de la paroi semi-rigide conformée (5) comporte une structure à saillie intérieure (14), agencée pour laisser libre ou ne pas occulter le passage de transfert (16) de contenu réalisé avec la structure à écartement radial, et remplir une fonction de positionnement axial fixe ou sensiblement fixe de la partie de fixation (8) de l'applicateur (7) dans la cavité de fixation (12a),
et optionnellement dans lequel la structure à saillie intérieure (14) comprend essentiellement, en particulier est constitué par, au moins une saillie (14a, 14b) prévue sur la face périphérique intérieure (18) de la portion de fixation (12) de la paroi semi-rigide conformée (5), dirigée vers l'axe (XX), agencée avec une localisation transversale et coopérant avec la face périphérique externe (17) et/ou l'une au moins des faces transversales d'extrémité (19) de la partie de fixation (8) de l'applicateur (7) de sorte à assurer le positionnement axial fixe ou sensiblement fixe de la partie de fixation (8) de l'applicateur (7) dans la cavité de fixation (12a).

10. Dispositif (1) selon la revendication 9, dans lequel une saillie de la structure à saillie intérieure (14) est localisée sur une partie seulement de la face périphérique intérieure (18) de la portion de fixation (12) entre ses deux bords (20) opposés ou est agencée de sorte que du contenu peut la traverser dans la direction de l'axe (XX) du dispositif (1), étant pourvue d'un passage traversant ou de pores ou étant formée de portions discontinues,
et optionnellement dans lequel la structure à saillie intérieure (14) comprend deux saillies, l'une (14a) vers le réservoir (2) de contenu et l'autre (14b) vers le moyen sécable (3), coopérant avec les deux faces transversales d'extrémité (19) de la partie de fixation (8) de l'applicateur (7) placée entre les deux saillies (14a, 14b).

11. Dispositif (1) selon l'une quelconque des revendications 1 à 10, dans lequel le moyen sécable (3) est choisi de type casser-tourner ou snap and twist,
et optionnellement dans lequel
l'applicateur (7) comporte une partie de fixation (8) telle qu'une tige, de forme générale cylindrique, et une partie d'application (9) telle qu'un pinceau, un tampon, une palette ou une tige, en particulier une partie d'application (9) adaptée pour une application précise quant à la taille, la forme, la limite, faible quant à la dimension, la surface, la quantité.

12. Dispositif (1) selon l'une quelconque des revendications 1 à 11, dans lequel la paroi semi-rigide conformée (5) est une paroi thermoformée et dans lequel la paroi de fermeture (6) est un opercule plan ou une seconde paroi thermoformée plane ou conformée en creux (10),
et optionnellement comprenant l'un ou plusieurs des caractéristiques suivante :, - à la paroi de fermeture (6) duquel est intégré ou rapporté de façon fixe un conteneur accessoire ou complémentaire (21a) pour un contenu accessoire ou complémentaire (21b), en particulier tel qu'un sachet flexible ;- la paroi semi-rigide conformée (5) comprend un second creux (22), distinct et espacé, fermé par une paroi de fermeture (6), de sorte à former un conteneur accessoire ou complémentaire pour un contenu accessoire ou complémentaire.

13. Dispositif (1) selon l'une quelconque des revendications 1 à 12, agencé avec un ou plusieurs autres dispositifs (1) analogues de sorte à former une plaque (23) de dispositifs (1), en particulier côte à côte, en particulier séparés les uns des autres par des moyens sécables.

14. Dispositif (1) selon l'une quelconque des revendications 1 à 13, une fois amené à l'état ouvert / dissocié pour son utilisation moyennant une opération d'ouverture / dissociation, par laquelle la partie sécable est rompue, comprenant deux parties dissociées l'une de l'autre :
- une partie d'utilisation (1a) qui comprend le réservoir (2) de contenu, la cavité de fixation (12a) de la partie de fixation (8) de l'applicateur (7) et l'applicateur (7) dont la partie d'application (9), libre et saillant de la portion de fixation (12) est découverte et peut être appliquée sur la surface d'application,
- une partie jetable (1b) qui comprend l'enceinte de protection (13a) dont la partie d'application (9) de l'applicateur (7) a été extraite et qui ne peut plus être remise en place sur la partie d'utilisation.

15. Dispositif (1) selon l'une quelconque des revendications 1 à 14, dans lequel pour la mise en oeuvre du dispositif (1) une fois amené à l'état ouvert / dissocié, le contenu est transféré du réservoir (2) de contenu à la partie d'application (9) de l'applicateur (7) moyennant, d'abord, une pression exercée brièvement sur la portion de réception (11) de la paroi semi-rigide conformée (5) pour amorcer le transfert, puis, le mouvement de la partie d'application (9) de l'applicateur (7) sur et au contact de la surface d'application.

## Patentansprüche

1. Vorrichtung (1) zur Aufnahme, zur Abgabe und zum Aufbringen eines Inhalts in Form einer Flüssigkeit, eines Gels, einer Creme, einer Paste, welche vor der Verwendung im geschlossenen Zustand/assoziiert ist und geeignet ist, für ihre Verwendung durch einen Öffnungs-/Dissoziationsvorgang in den offenen Zustand geführt/dissoziiert zu werden auf eine Applikationsfläche, mit einer Achse (XX), umfassend eine konforme halbstarre Wand (5), eine Verschlusswand (6) und einen Applikator (7) mit einem Befestigungsteil (8) und einem Applikationsteil (9) in gegenseitiger Verlängerung, wobei:
- die konforme halbstarre Wand (5) umfasst einen ebenen Umfangsrand (4) und einen Hohlraum (10), der an einer Seite der Ebene (P) des Rands (4) angeordnet und von diesem umgeben ist, mit, in gegenseitiger Verlängerung und Verbindung, einem Aufnahmeabschnitt (11) des Inhalts, der durch einen äußeren Druck verformbar ist, einem Befestigungsabschnitt (12) mit einem inneren Vorsprung und einem Schutzabschnitt (13), die jeweils mit dem Befestigungsteil (8) und dem Applikationsteil (9) des Applikators (7) zusammenwirken,
- die Verschlusswand (6) die konforme halbstarre Wand (5) bedeckt, starr am Rand (4) befestigt ist und den Hohlraum (10) hermetisch verschließt, indem ein Reservoir (2) des Inhalts, ein Befestigungshohlraum (12a) des Befestigungsteils (8) des Applikators (7) und eine Schutzhülle (13a) des Applikationsteils (9) des Applikators (7) aufgenommen werden, der geeignet ist, beim Öffnungs-/Dissoziationsvorgang von diesem Applikationsteil (9) dissoziiert zu werden, um diesen freizugeben,
- ein abtrennbares Mittel (3) der konformen halbstarren Wand (5) und der Verschlusswand (6) quer zwischen dem Befestigungsabschnitt (12) und dem Schutzabschnitt (13) angeordnet ist und geeignet ist, für den Öffnungs-/Dissoziationsvorgang abgebrochen zu werden,
- eine Transferpassage (16) des Inhalts sich entlang der Achse (XX) zwischen und in Verbindung mit dem Reservoir (2) des Inhalts und dem Applikationsteil (9) des Applikators (7) derart erstreckt, dass für die Verwendung der Vorrichtung (1) im offenen/dissozierten Zustand Inhalt von dem Reservoir (2) des Inhalts zu dem Applikationsteil (9) des Applikators (7) in Folge eines äußeren Drucks auf den Aufnahmeabschnitt (11) der konformen halbstarren Wand (5) transferiert wird,
**dadurch gekennzeichnet, dass** die Transferpassage (16) des Inhalts mit einer Struktur mit einem radialen Abstand ausgeführt ist, der ein Fehlen einer peripheren Dichtheit zwischen der äußeren Umfangsfläche (17) des Befestigungsteils (8) des Applikators (7) und der inneren Umfangsfläche (18) in Bezug auf den Befestigungsabschnitt (12) der konformen halbstarren Wand (5) gewährleistet, wobei sich die Struktur mit dem radialen Abstand kommunizierend und kontinuierlich entlang der Achse (XX) auf der einen Seite zwischen dem Reservoir (2) des Inhalts und auf der anderen Seite dem abtrennbaren Mittel (3) und dem Applikationsteil (9) des Applikators (7) erstreckt, und dass die Zufuhr von Inhalt, der von dem Applikationsteil (9) des Applikators (7) aufzubringen ist, aus seiner äußeren Umfangshülle (9a) durchgeführt wird, wenn der Inhalt geeignet ist, die Transferpassage (16) durchqueren und wie mit einem Pinsel aufgebracht werden zu können.

2. Vorrichtung (1) nach Anspruch 1, wobei der Befestigungsteil (8) des Applikators (7) derart angeordnet ist, dass Inhalt diesen nicht entlang der Achse (XX) durchqueren kann, wobei insbesondere dieser Befestigungsteil (8) voll oder frei von einem durchquerenden Kanal oder von Poren ist.

3. Vorrichtung (1) nach einem der Ansprüche 1 und 2, wobei die Transferpassage (16) des Inhalts mit einer Struktur mit einem radialen Abstand ferner zwischen der äußeren Umfangsfläche (17) des Befestigungsteils (8) des Applikators (7) und der Innenfläche (6a) in Bezug auf die Verschlusswand (6) ausgeführt ist.

4. Vorrichtung (1) nach einem der Ansprüche 1 bis 3, wobei die Transferpassage (16) des Inhalts im Wesentlichen mindestens einen Kanal und insbesondere mehrere Kanäle (16a, 16b) umfasst, insbesondere daraus besteht, die winkelig beabstandet sind, axial angeordnet sind, von der äußeren Umfangsfläche (17) des Befestigungsteils (8) des Applikators (7) und der inneren Umfangsfläche (18) in Bezug auf den Befestigungsabschnitt (12) der konformen halbstarren Wand (5) durch die Struktur mit radialem Abstand begrenzt werden, die sich kommunizierend und kontinuierlich erstreckt und auf einer Seite in das Reservoir (2) des Inhalts und auf der anderen Seite zu der äußeren Umfangshülle (9a), insbesondere an diese angrenzend, des Applikationsteils (9) des Applikators (7) mündet, der zu dem Befestigungsteil (8) des Applikators (7) angeordnet ist, insbesondere auf der Außenfläche (9b) des Applikationsteils (9) des Applikators (7), und gegebenenfalls
umfassend mehrere Kanäle (16a, 16b), die mindestens im Wesentlichen symmetrisch in Bezug auf eine Mittelebene (Q) der Vorrichtung (1), orthogonal zur Ebene (P) des Umfangsrands (4) der konformen halbstarren Wand (5) angeordnet sind, und/oder umfassend mindestens einen Kanal, der derart eingerichtet ist, dass er angeordnet wird, indem er entweder radial am weitesten beabstandet ist oder radial am wenigsten von der Verschlusswand (6) beabstandet ist, und insbesondere umfassend mindestens zwei Kanäle, die eingerichtet sind, derart angeordnet zu werden, dass der eine radial am weitesten beabstandet ist und der andere radial am wenigsten von der Verschlusswand (6) beabstandet ist.

5. Vorrichtung (1) nach Anspruch 4, wobei ein Kanal (16a, 16b) im Querschnitt eine abgeflachte Form aufweist, die sich in Umfangsrichtung von der äußeren Umfangsfläche (17) des Befestigungsteils (8) des Applikators (7) und von der inneren Umfangsfläche (18) in Bezug auf den Befestigungsabschnitt (12) der konformen halbstarren Wand (5) erstreckt, und wobei gegebenenfalls
ein Kanal (16a, 16b), zu der äußeren Umfangshülle (9a) des Applikationsteils (9) des Applikators (7) hin, einen verbreiterten Querschnitt in der Umfangsrichtung der äußeren Umfangsfläche (17) des Befestigungsteils (8) des Applikators (7) und der inneren Umfangsfläche (18) in Bezug auf den Befestigungsabschnitt (12) der konformen halbstarren Wand (5) aufweist, um mindestens 20 %, insbesondere mindestens 40 %, spezifischer mindestens 60 %, und gegebenenfalls die Gesamtheit oder nahezu die Gesamtheit der Umfangsausdehnung der äußeren Umfangshülle (9a) des Applikationsteils (9) des Applikators (7) oder der Außenfläche des Applikationsteils (9) des Applikators (7) zu repräsentieren.

6. Vorrichtung (1) nach einem der Ansprüche 4 oder 5, wobei:
- der Befestigungsabschnitt (12) der konformen halbstarren Wand (5), mindestens in seinem mittleren longitudinalen Teil, insbesondere über seine gesamte oder im Wesentlichen gesamte Länge, einen Innenquerschnitt mit einer allgemeinen Form eines U, eines U mit auseinanderlaufenden Verzweigungen, eines V oder einer Halbkreisform aufweist,
- der Befestigungshohlraum (12a), mindestens in seinem mittleren longitudinalen Teil, insbesondere über seine gesamte oder im Wesentlichen gesamte Länge, einen Innenquerschnitt mit einer allgemeinen Form eines Quadrats, Rechtecks, Trapezes mit der großen Basis zu der Verschlusswand (6) hin oder mit einer Form eines Halbkreisbogens aufweist,
- die Form des Innenquerschnitts des Befestigungsabschnitts (12) der konformen halbstarren Wand (5) und die Form des Innenquerschnitts des Befestigungshohlraums (12a) der Form des Außenquerschnitts des Befestigungsteils (8) des Applikators (7) entsprechen, um den mindestens einen Kanal der Transferpassage (16) des Inhalts mit der Struktur mit radialem Abstand zu bilden,
und wobei gegebenenfalls
- der Befestigungsteil (8) des Applikators (7), mindestens in seinem mittleren longitudinalen Teil, insbesondere über seine gesamte oder im Wesentlichen gesamte Länge, einen Außenquerschnitt mit einer allgemeinen polygonalen oder gekrümmten, insbesondere elliptischen, Form aufweist,
- wobei die Form des Außenquerschnitts des Befestigungsteils (8) des Applikators (7) der Form des Innenquerschitts des Befestigungsabschnitts (12) der konformen halbstarren Wand (5) und der Form des Innenquerschnitts des Befestigungshohlraums (12a) entspricht, um den mindestens einen Kanal der Transferpassage (16) des Inhalts mit der Struktur mit radialem Abstand zu bilden.

7. Vorrichtung (1) nach einem der Ansprüche 1 bis 6, welche die Struktur mit radialem Abstand und eine Struktur mit Kontakt oder Quasi-Kontakt (15) zwischen der äußeren Umfangsfläche (17) des Befestigungsteils (8) des Applikators (7) und der inneren Umfangsfläche (18) in Bezug auf den Befestigungsabschnitt (12) der konformen halb-starren Wand (5) kombiniert, welche eingerichtet ist, um die Transferpassage (16) des Inhalts frei zu lassen oder nicht zu blockieren, die mit der Struktur mit radialem Abstand ausgeführt ist, und eine Funktion der festen oder im Wesentlichen festen radialen Positionierung des Befestigungsteils (8) des Applikators (7) in dem Befestigungshohlraum (12a) zu erfüllen.

8. Vorrichtung (1) nach einem der Ansprüche 1 bis 7, wobei:
- der Befestigungsabschnitt (12) der konformen halbstarren Wand (5), mindestens in seinem mittleren longitudinalen Teil, insbesondere über seine gesamte oder im Wesentlichen gesamte Länge, einen Innenquerschnitt mit einer allgemeinen Form eines U, eines U mit auseinanderlaufenden Verzweigungen, eines V oder einer Halbkreisform aufweist,
- der Befestigungshohlraum (12a), mindestens in seinem mittleren longitudinalen Teil, insbesondere über seine gesamte oder im Wesentlichen gesamte Länge, einen Innenquerschnitt mit einer allgemeinen Form eines Quadrats, Rechtecks, Trapezes mit der großen Basis zu der Verschlusswand (6) hin oder mit einer Form eines Halbkreisbogens aufweist,
- der Befestigungsteil (8) des Applikators (7), mindestens in seinem mittleren longitudinalen Teil, insbesondere über seine gesamte oder im Wesentlichen gesamte Länge, einen Außenquerschnitt mit einer allgemeinen polygonalen oder gekrümmten, insbesondere elliptischen, Form aufweist,
- die Form des Außenquerschnitts des Befestigungsteils (8) des Applikators (7) der Form des Innenquerschnitts des Befestigungsabschnitts (12) der konformen halbstarren Wand (5) und der Form des Innenquerschnitts des Befestigungshohlraums (12a) entspricht, um die Struktur mit Kontakt oder Quasi-Kontakt (15) zu bilden, wobei ein Kontakt oder ein Quasi-Kontakt zwischen der äußeren Umfangsfläche (17) des Befestigungsteils (8) des Applikators (7) und der inneren Umfangsfläche (18) in Bezug auf den Befestigungsabschnitt (12) der konformen halbstarren Wand (5) gewährleistet wird, wobei der Kontakt oder ein Quasi-Kontakt eingerichtet ist, die Struktur mit radialem Abstand aufzunehmen.

9. Vorrichtung (1) nach einem der Ansprüche 1 bis 8, wobei der Befestigungsabschnitt (12) der konformen halbstarren Wand (5) eine Struktur mit einem inneren Vorsprung (14) umfasst, welche eingerichtet ist, um die Transferpassage (16) des Inhalts frei zu lassen oder nicht zu blockieren, die mit der Struktur mit radialem Abstand ausgeführt ist, und eine Funktion der festen oder im Wesentlichen festen axialen Positionierung des Befestigungsteils (8) des Applikators (7) in dem Befestigungshohlraum (12a) zu erfüllen,
und wobei gegebenenfalls die Struktur mit einem inneren Vorsprung (14) im Wesentlichen mindestens einen Vorsprung (14a, 14b) umfasst, insbesondere daraus besteht, der auf der inneren Umfangsfläche (18) des Befestigungsabschnitts (12) der konformen halbstarren Wand (5) gebildet ist, der zur Achse (XX) gerichtet ist, der mit einer transversalen Lokalisierung eingerichtet ist und mit der äußeren Umfangsfläche (17) und/oder mindestens einer der transversalen Endflächen (19) des Befestigungsteils (8) des Applikators (7) zusammenwirkt, um die feste oder im Wesentlichen feste axiale Positionierung des Befestigungsteils (8) des Applikators (7) in dem Befestigungshohlraum (12a) zu gewährleisten.

10. Vorrichtung (1) nach Anspruch 9, wobei ein Vorsprung der Struktur mit einem inneren Vorsprung (14) nur auf einem Teil der inneren Umfangsfläche (18) des Befestigungsabschnitts (12) zwischen seinen beiden gegenüberliegenden Rändern (20) angeordnet ist oder derart eingerichtet ist, dass Inhalt in der Richtung der Achse (XX) der Vorrichtung (1) diesen durchqueren kann, wobei er mit einer Querpassage oder Poren versehen ist oder mit diskontinuierlichen Teilen ausgebildet ist,
und wobei gegebenenfalls die Struktur mit einem inneren Vorsprung (14) zwei Vorsprünge umfasst, einen (14a) zu dem Reservoir (2) des Inhalts hin und einen anderen (14b) zu dem abtrennbaren Mittel (3) hin, wobei sie mit den beiden transversalen Endflächen (19) des Befestigungsteils (8) des Applikators (7) zusammenwirken, der zwischen den beiden Vorsprüngen (14a, 14b) angeordnet ist.

11. Vorrichtung (1) nach einem der Ansprüche 1 bis 10, wobei das abtrennbare Mittel (3) vom Typ eines Abbrechens durch Drehen oder Snap-und-Twist gewählt ist,
und wobei gegebenenfalls
der Applikator (7) einen Befestigungsteil (8) umfasst, wie einen Stiel, mit allgemein zylindrischer Form, und einen Applikationsteil (9), wie einen Pinsel, ein Kissen, eine Palette oder einen Stil, insbesondere einen Applikationsteil (9), der für ein präzises Aufbringen hinsichtlich der Größe, der Form, der Grenze, ein geringes Aufbringen hinsichtlich der Abmessung, der Fläche, der Menge, geeignet ist.

12. Vorrichtung (1) nach einem der Ansprüche 1 bis 11, wobei die konforme halbstarre Wand (5) eine warmgeformte Wand ist, und wobei die Verschlusswand (6) ein ebener Deckel oder eine zweite ebene warmgeformte Wand oder zum Hohlraum (10) konform ist,
und gegebenenfalls umfassend eines oder mehrere der folgenden Merkmale: - in der Verschlusswand (6), in der fest ein zusätzlicher oder komplementärer Behälter (21 a) für einen zusätzlichen oder komplementären Inhalt (21 b), wie insbesondere ein flexibler Beutel, integriert oder aufgenommen ist; - die konforme halbstarre Wand (5) umfasst einen zweiten verschiedenen und beabstandeten Hohlraum (22), der von einer Verschlusswand (6) verschlossen wird, um einen zusätzlichen oder komplementären Behälter für einen zusätzlichen oder komplementären Inhalt zu bilden.

13. Vorrichtung (1) nach einem der Ansprüche 1 bis 12, welche mit einer oder mehreren anderen analogen Vorrichtungen (1) angeordnet ist, um eine Platte (23) von Vorrichtungen (1), insbesondere Seite an Seite, insbesondere durch abtrennbare Mittel voneinander getrennt, zu bilden.

14. Vorrichtung (1) nach einem der Ansprüche 1 bis 13, welche, sobald sie für ihre Verwendung durch einen Öffnungs /Dissoziationsvorgang in den offenen Zustand gebracht/dissoziiert wurde, durch den der abtrennbare Teil zerbrochen wurde, zwei voneinander dissoziierte Teile umfasst:
- einen Verwendungsteil (1 a), der das Reservoir (2) des Inhalts, den Befestigungshohlraum (12a) des Befestigungsteils (8) des Applikators (7) und den Applikator (7) umfasst, dessen Applikationsteil (9), frei und vorspringend von dem Befestigungsabschnitt (12), freigelegt ist und auf die Applikationsfläche aufgebracht werden kann,
- einen wegwerfbaren Teil (1b), der die Schutzhülle (13a) umfasst, aus welcher der Applikationsteil (9) des Applikators (7) entfernt wurde, und der nicht mehr auf den Verwendungsteil platziert werden kann.

15. Vorrichtung (1) nach einem der Ansprüche 1 bis 14, wobei zur Verwendung der Vorrichtung (1), sobald sie in den offenen Zustand gebracht/dissoziiert wurde, der Inhalt von dem Reservoir (2) des Inhalts zu dem Applikationsteil (9) des Applikators (7) transferiert wird, indem zuerst ein kurzer Druck auf den Aufnahmeteil (11) der konformen halbstarren Wand (5) ausgeübt wird, um den Transfer zu fördern, und dann der Applikationsteil (9) des Applikators (7) auf und in Kontakt mit der Applikationsfläche bewegt wird.

## Claims

1. Device (1) for containing, dispensing, and applying to an application surface a content in liquid, gel, cream, or paste form, in the closed/associated state prior to use, able to be placed in the open/separated state for use by means of an opening/separation operation, comprising, with an axis (XX), a shaped semi-rigid wall (5), a closing wall(6), and an applicator (7) having an attachment portion (8) and an application portion (9) that are extensions of one another, such that:
▪ the shaped semi-rigid wall (5) comprises a flat peripheral edge (4) and a recess (10) located on one side of the plane (P) of the edge (4) and surrounded by it, having, as extensions of one another and in communication, a content receiving portion (11) that is deformable by means of external pressure, an attachment part (12) with inner projection and a protection portion (13) respectively engaging with the attachment portion (8) and the application portion (9) of the applicator (7),
▪ the closing wall (6) covers the shaped semi-rigid wall (5), is rigidly secured to the edge (4), and seals the recess (10) to provide a content (2) vessel, an attachment cavity (12a) for the attachment portion (8) of the applicator (7), and a protective enclosure (13a) for the application portion (9) of the applicator (7) which is adapted, during the opening/separation operation, to separate from said application portion (9) in order to expose it,
▪ a breakable means (3) of the shaped semi-rigid wall (5) and of the closing wall (6) is located transversely between the attachment part (12) and the protection portion (13), and is adapted to break for the opening/separation operation,
▪ a content-transfer passage (16) extends along the axis (XX) between, and in communication with, the content (2) vessel and the application portion (9) of the applicator (7), so that, in order to use the device (1) in the open/separated state, content is transferred from the content (2) vessel to the application portion (9) of the applicator (7) when external pressure is exerted on the receiving portion (11) of the shaped semi-rigid wall (5),
**characterized in that** the content-transfer passage (16) is made with a structure having radial spacing that ensures there is no peripheral sealing between the outer peripheral surface (17) of the attachment portion (8) of the applicator (7) and the facing inner peripheral surface (18) of the attachment part (12) of the shaped semi-rigid wall (5), the structure with radial spacing extending in a communicating and continuous manner along the axis (XX), between the content (2) vessel on the one side, and the breakable means (3) and application portion (9) of the applicator (7) on the other, and **in that** the content to be applied from the application portion (9) of the applicator (7) is supplied from its outer peripheral shell (9a), while the content is capable of passing through the transfer passage (16) and of being applied as though with a brush.

2. Device (1) according to claim 1, wherein the attachment portion (8) of the applicator (7) is arranged so that the content cannot pass through it along the axis (XX), in particular wherein said attachment portion (8) is solid or without any through-channel or pores.

3. Device (1) according to any one of claims 1 and 2, wherein the content-transfer passage (16) is made with a structure having radial spacing between, in addition, the outer peripheral surface (17) of the attachment portion (8) of the applicator (7) and the facing inner surface (6a) of the closing wall (6).

4. Device (1) according to any one of claims 1 to 3, wherein the content-transfer passage (16) essentially comprises, in particular consists of, at least one channel, and in particular a plurality of channels (16a, 16b) angularly spaced apart, arranged axially, delimited by the outer peripheral surface (17) of the attachment portion (8) of the applicator (7) and the facing inner peripheral surface (18) of the attachment part (12) of the shaped semi-rigid wall (5), by means of the structure with radial spacing, extending in a communicating and continuous manner and opening on one side into the content (2) vessel and on the other side near, in particular adjacent to, the outer peripheral shell (9a) of the application portion (9) of the applicator (7) located near the attachment portion (8) of the applicator (7), in particular on the outer surface (9b) of the application portion (9) of the applicator (7),
and optionally comprising a plurality of channels (16a, 16b) arranged at least substantially symmetrically with respect to a median plane (Q) of the device (1), orthogonal to the plane (P) of the peripheral edge (4) of the shaped semi-rigid wall (5), and/or comprising at least one channel arranged so as to be positioned either the furthest away radially or the closest radially to the closing wall (6), and in particular comprising at least two channels arranged so that one is positioned the furthest away radially and the other the closest radially to the closing wall (6).

5. Device (1) according to claim 4, wherein the cross-section of a channel (16a, 16b) has a shape that is flattened in the peripheral direction of the outer peripheral surface (17) of the attachment portion (8) of the applicator (7) and of the facing inner peripheral surface (18) of the attachment part (12) of the shaped semi-rigid wall (5),
and optionally wherein a channel (16a, 16b) has, near the outer peripheral shell (9a) of the application portion (9) of the applicator (7), a section enlarged in the peripheral direction of the outer peripheral surface (17) of the attachment portion (8) of the applicator (7) and of the facing inner peripheral surface (18) of the attachment part (12) of the shaped semi-rigid wall (5), so as to represent at least 20%, in particular at least 40%, more particularly at least 60%, and possibly all or substantially all of the peripheral extent of the outer peripheral shell (9a) of the application portion (9) of the applicator (7) or of the outer surface of the application portion (9) of the applicator (7).

6. Device (1) according to any one of claims 4 or 5, wherein:
- the attachment part (12) of the shaped semi-rigid wall (5) has, at least in its middle longitudinal section, in particular along all or substantially all of its length, an internal cross-section in the general shape of a U, a U with diverging arms, a V, or a semi-circular shape,
- the attachment cavity (12a) has, at least in its middle longitudinal section, in particular along all or substantially all of its length, an internal cross-section in the general shape of a square, rectangle, trapezium with its large base towards the closing wall (6), or a sector of a semi-circle,
- the shape of the internal cross-section of the attachment part (12) of the shaped semi-rigid wall (5) and the shape of the internal cross-section of the attachment cavity (12a) correspond to the shape of the external cross-section of the attachment portion (8) of the applicator (7), to form the at least one channel (16) of the content-transfer passage, with the structure having radial spacing,
and optionally wherein:
- the attachment portion (8) of the applicator (7) has, at least in its middle longitudinal section, in particular along all or substantially all of its length, an external cross-section of a shape that is generally polygonal or curved, in particular elliptical,
- the shape of the external cross-section of the attachment portion (8) of the applicator (7) corresponds to the shape of the internal cross-section of the attachment part (12) of the shaped semi-rigid wall (5) and the shape of the internal cross-section of the attachment cavity (12a), to form the at least one channel of the content-transfer passage (16), with the structure having radial spacing.

7. Device (1) according to any one of claims 1 to 6, which combines the structure having radial spacing and a structure having contact or near-contact (15) between the outer peripheral surface (17) of the attachment portion (8) of the applicator (7) and the facing inner peripheral surface (18) of the attachment part (12) of the shaped semi-rigid wall (5), arranged to leave open or unobstructed the content-transfer passage (16) formed with the structure having radial spacing, and to fulfill a function of fixed or substantially fixed radial positioning of the attachment portion (8) of the applicator (7) within the attachment cavity (12a).

8. Device (1) according to any one of claims 1 to 7, wherein:
- the attachment part (12) of the shaped semi-rigid wall (5) has, at least in its middle longitudinal section, in particular along all or substantially all of its length, an internal cross-section in the general shape of a U, a U with diverging arms, a V, or a semi-circular shape,
- the attachment cavity (12a) has, at least in its middle longitudinal section, in particular along all or substantially all of its length, an internal cross-section in the general shape of a square, rectangle, trapezium with its large base towards the closing wall (6), or a sector of a semi-circle,
- the attachment portion (8) of the applicator (7) has, at least in its middle longitudinal section, in particular along all or substantially all of its length, an external cross-section of a shape that is generally polygonal or curved, in particular elliptical,
- the shape of the external cross-section of the attachment portion (8) of the applicator (7) corresponds to the shape of the internal cross-section of the attachment part (12) of the shaped semi-rigid wall (5) and the shape of the internal cross-section of the attachment cavity (12a), to form the structure having contact or near-contact (15), ensuring contact or near-contact between the outer peripheral surface (17) of the attachment portion (8) of the applicator (7) and the facing inner peripheral surface (18) of the attachment part (12) of the shaped semi-rigid wall (5), the contact or near-contact being arranged so as to provide the structure having radial spacing.

9. Device (1) according to any one of claims 1 to 8, wherein the attachment part (12) of the shaped semi-rigid wall (5) has an internally projecting structure (14) arranged to leave open or unobstructed the content-transfer passage (16) formed with the structure having radial spacing, and to fulfill a function of fixed or substantially fixed axial positioning of the attachment portion (8) of the applicator (7) within the attachment cavity (12a),
and optionally wherein the internally projecting structure (14) essentially comprises, in particular consists of, at least one projection (14a, 14b) provided on the inner peripheral surface (18) of the attachment part (12) of the shaped semi-rigid wall (5), directed towards the axis (XX), arranged with a transverse location and engaging with the outer peripheral surface (17) and/or at least one of the transverse end faces (19) of the attachment portion (8) of the applicator (7) so as to ensure the fixed or substantially fixed axial positioning of the attachment portion (8) of the applicator (7) within the attachment cavity (12a).

10. Device (1) according to claim 9, wherein a projection of the internally projecting structure (14) is located on only a portion of the inner peripheral surface (18) of the attachment part (12) between its two opposing edges (20) or is arranged so that content can pass through it in the direction of the axis (XX) of the device (1), it being provided with a through-passage or pores or being formed of discontinuous portions,
and optionally
wherein the internally projecting structure (14) comprises two projections, one (14a) near the content (2) vessel and the other (14b) near the breakable means (3), engaging with the two transverse end faces (19) of the applicator (7) attachment portion (8) positioned between the two projections (14a, 14b).

11. Device (1) according to one of claims 1 to 10, wherein the breakable means (3) is of the snap and twist type,
and optionally
wherein the applicator (7) comprises an attachment portion (8) such as a rod, generally cylindrical in shape, and an application portion (9) such as a brush, a pad, a spreader, or a wand, in particular an application portion (9) suitable for an application that is precise in size, shape, and limits, and that is small in dimensions, surface area, and amount.

12. Device (1) according to any one of claims 1 to 11, wherein the shaped semi-rigid wall (5) is a thermoformed wall and wherein the closing wall (6) is a flat coverplate or a second thermoformed wall that is flat or with one or more recesses (10),
and optionally comprising one or more of the following features
- the closing wall (6) has an integrated or fixedly related secondary or supplemental container (21 a) for secondary or supplemental content (21 b), in particular such as a flexible bag,
- the shaped semi-rigid wall (5) comprises a second recess (22), separate and spaced apart, closed off by a closing wall (6), so as to form an secondary or supplemental container for secondary or supplemental content.

13. Device (1) according to any one of claims 1 to 12, arranged with one or more other similar devices (1) so as to form a sheet (23) of devices (1), in particular side by side, in particular separated from each other by breakable means.

14. Device (1) according to any one of claims 1 to 13, placed in the open/separated state for use, by means of an opening/separation operation whereby the breakable portion is broken, comprising two portions separated from one another:
- a usage portion (1 a) which comprises the content vessel (2), the attachment cavity (12a) for the attachment portion (8) of the applicator (7), and the applicator (7) of which the application portion (9), exposed and protruding from the attachment part (12), is uncovered and can be applied to the application surface,
- a disposable portion (1 b) which comprises the protective enclosure (13a) from which the application portion (9) of the applicator (7) has been extracted and which cannot be placed back on the usage portion.

15. Device (1) according to any of claims 1 to 14, wherein to make use of the device (1) once it is in the open/separated state, the content is transferred from the content vessel (2) to the application portion (9) of the applicator (7) by means of, firstly, a pressure exerted briefly on the receiving portion (11) of the shaped semi-rigid wall (5) to initiate the transfer, then the movement of the application portion (9) of the applicator (7) on and in contact with the application surface.
